(19) Europäisches Patentamt
European Patent Office
Office européen des brevets

(11) Veröffentlichungsnummer : **0 386 557 B1**

# (12) EUROPÄISCHE PATENTSCHRIFT

(45) Veröffentlichungstag der Patentschrift :
**10.06.92 Patentblatt 92/24**

(51) Int. Cl.$^5$ : **C07D 405/06,** C07D 405/14, A01N 43/50, A01N 43/653

(21) Anmeldenummer : **90103616.0**

(22) Anmeldetag : **24.02.90**

## (54) Verfahren zur Bekämpfung von Pilzen ausserhalb des menschlichen oder tierischen Körpers.

Die Akte enthält technische Angaben, die nach dem Eingang der Anmeldung eingereicht wurden und die nicht in dieserPatentschrift enthalten sind.

(30) Priorität : **10.03.89 DE 3907729**

(43) Veröffentlichungstag der Anmeldung :
**12.09.90 Patentblatt 90/37**

(45) Bekanntmachung des Hinweises auf die Patenterteilung :
**10.06.92 Patentblatt 92/24**

(84) Benannte Vertragsstaaten :
**AT BE CH DE DK ES FR GB GR IT LI NL SE**

(56) Entgegenhaltungen :
**EP-A- 0 332 073**
**DE-A- 3 218 129**
**DE-A- 3 218 130**
**DE-A- 3 511 411**
**DE-A- 3 737 888**

(73) Patentinhaber : **BASF Aktiengesellschaft**
**Carl-Bosch-Strasse 38**
**W-6700 Ludwigshafen (DE)**

(72) Erfinder : **Karbach, Stefan, Dr.**
**Grundwiesenweg 44**
**W-6730 Neustadt (DE)**
Erfinder : **Seele, Rainer, Dr.**
**Leiblstrasse 3**
**W-6701 Fussgoenheim (DE)**
Erfinder : **Wegner, Guenter, Dr.**
**Paul-Egell-Strasse 7**
**W-6720 Speyer (DE)**
Erfinder : **Smuda, Hubert, Dr.**
**Bierhelderweg 7**
**W-6900 Heidelberg (DE)**
Erfinder : **Bireckoven, Bernd, Dr.**
**Hoelderlinstasse 19**
**W-7408 Kusterdingen (DE)**
Erfinder : **Lorenz, Gisela, Dr.**
**Erlenweg 13**
**W-6730 Neustadt (DE)**
Erfinder : **Ammermann, Eberhard, Dr.**
**Sachsenstrasse 3**
**W-6700 Ludwigshafen (DE)**

EP 0 386 557 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Bekämpfung von phytopathogene Pilzen.

Aus der EP-A-94 564 sind Azolylmethyloxirane, insbesondere das 2-(1,2,4-Triazol-1-yl-methyl)-2-(4-chlorphenyl)-3-(3-trifluormethyl)-oxiran bekannt, dessen Wirkung als Fungizid nicht in allen Fällen befriedigt. Aus DE-A-3 218 130, 3 218 129 und 3 511 411 sind Azolylmethyloxirane als Fungizide bekannt, die Trifluormethylphenylreste enthalten.

Der Erfindung lag daher die Aufgabe zugrunde Azolylmethyloxirane mit verbesserter Wirkung zur Verfügung zu stellen.

Es wurde nun gefunden, daß Verbindungen der Formel I

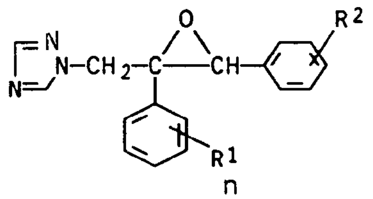

I,

in der

$R^1$ Wasserstoff, Halogen, Nitro, Phenyl, Phenoxy, $C_1$-$C_5$-Alkyl, $C_1$-$C_5$-Alkoxy, $C_1$-$C_4$-Halogenalkyl, $C_1$-$C_5$-Halogenalkoxy und darüber hinaus $R^1$ zusammen mit dem Phenylkern auch einen Naphthylrest bilden kann, der unsubstituiert oder durch $R^1$ substituiert ist,

n eine ganze Zahl von 1 bis 5,

$R^2$ ortho-Trifluormethyl,

bedeuten, oder der Verbindungen der Formel I, in der $R^1$ Fluor, Brom, Nitro, Phenyl, Phenoxy, $C_1$-$C_5$-Alkyl, $C_1$-$C_5$-Alkoxy, $C_1$-$C_4$-Halogenalkyl, $C_1$-$C_5$-Halogenalkoxy und darüber hinaus $R^1$ zusammen mit dem Phenylkern auch einen Naphthylrest bilden kann, der unsubstituiert oder durch $R^1$ substituiert ist,

n eine ganze Zahl von 1 bis 5,

$R^2$ Para-Trifluormethyl,

bedeuten, und deren für Pflanzen verträgliche Säureadditionssalze oder Metallsalze eine bessere fungizide Wirkung, insbesondere gegen Getreidekrankheiten, besitzen als bekannte Azolylmethyloxirane.

Die Verbindungen der Formel I enthalten chirale Zentren und werden im allgemeinen in Form von Racematen bzw. als Diastereomerengemische von erythro- bzw. threo-Formen erhalten. Die erythro- bzw. threo-Dieasteromeren lassen sich bei den erfindungsgemäßen Verbindungen in üblicher Weise, beispielsweise aufgrund ihrer unterschiedlichen Löslichkeit oder durch Säulenchromatographie trennen und in reiner Form isolieren. Aus einem solchen isolierten Diastereomeren kann man mit bekannten Methoden einheitliche Enantiomere erhalten. Als fungizide Mittel werden sowohl die einheitlichen Diastereomere bzw. Enantiomere wie auch deren bei der Synthese anfallende Gemische verwendet.

$R^1$ bedeutet beispielsweise:

Halogen, wie Fluor, Chlor, Brom oder Jod, Nitro, Phenyl oder Phenoxy, z.B. in para-Stellung, $C_1$-$C_5$-Alkyl oder $C_1$-$C_5$-Alkoxi, wobei der Alkylrest jeweils für Methyl, Ethyl, Propyl, Isopropyl, n-Butyl, sec.-Butyl, tert.-Butyl, n-Pentyl oder Neopentyl steht,

$C_1$-$C_4$-Halogenalkyl oder $C_1$-$C_4$-Halogenalkoxy, wobei insbesondere einfach bis perhalogenierte Alkylreste wie $CH_2Cl$, $CHCl_2$, $CCl_3$, $CH_2F$, $CHF_2$, $CH_2Br$, $CHBrCl$, $CF_2Cl$, $C_2F_5$, $CF_2$-$CHF_2$, $CH_2CH_2Cl$, $CH_2CHCl_2$, $CH_2CH_2Br$, $C_3F_7$ und insbesondere $CF_3$ zu nennen sind.

Darüber hinaus kann $R^1$ zusammen mit dem Phenylkern, an das es gebunden ist, einen Naphthylrest, z.B. 1-Naphthyl oder 2-Naphthyl, bilden der wiederum ggf. ein- oder mehrfach durch die oben für $R^1$ genannten Reste substituiert ist.

n steht für eine ganze Zahl von 1 bis 5, insbesondere 1 bis 3.

Säureadditionssalze sind beispielsweise die Hydrochloride, Hydrobromide, Sulfate, Nitrate, Phosphate, Oxalate, oder Dodecylbenzolsulfonate. Die Wirksamkeit der Salze geht auf das Kation zurück, so daß es auf das Anion i. a. nicht ankommt. Die erfindungsgemäßen Wirkstoffsalze werden hergestellt durch Umsetzung der Azolylmethyloxirane (I) mit geeigneten Säuren.

Metallsalze der Wirkstoffe I oder ihrer Salze können z.B. mit Kupfer, Zink, Zinn, Mangan, Eisen, Kobalt oder Nickel gebildet werden, indem man die Azolylmethyloxirane mit entsprechenden Metallsalzen umsetzt.

Die Verbindungen der Formel I können hergestellt werden, indem man

a) eine Verbindung der Formel II

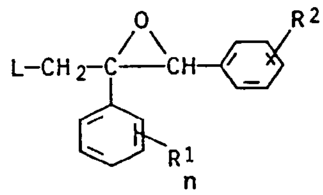

II,

in welcher

$R^1$ und $R^2$ die angegebenen Bedeutungen haben und L eine nucleophil substituierbare Abgangsgruppe darstellt, mit einer Verbindung der Formel III

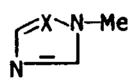

III,

in der Me ein Wasserstoffatom oder ein Metallatom bedeutet und X die angegebene Bedeutung hat, zur Umsetzung bringt, oder

b) eine Verbindung der Formel IV,

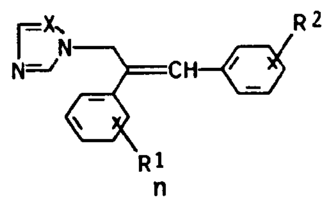

IV

in welcher $R^1$, $R^2$, und n die angegebenen Bedeutungen haben, und X N ist, in die Epoxide überführt und die so erhaltenen Verbindungen gegebenenfalls in ihre Salze mit für Pflanzen verträglichen Säuren überführt.

Die Reaktion a) erfolgt - falls Me ein Wasserstoffatom bedeutet - gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers.

Zu den bevorzugten Lösungs- und Verdünnungsmitteln gehören Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ester wie Essigsäureethylester, Essigsäuremethylester oder Essigsäurebutylester, Ether wie Tetrahydrofuran, Diethylether, Methyl-tert.-butylether, Dimethoxyethan, Dioxan oder Diisopropylether, Amide wie Dimethylformamid, Dimethylacetamid oder N-Methylpyrrolidon, ferner Dimethylsulfoxid, Sulfolan oder entsprechende Gemische.

Geeignete Basen, die gegebenenfalls auch als säurebindende Mittel bei der Reaktion verwendet werden können, sind beispielsweise Alkalihydroxid wie Lithium-, Natrium- oder Kaliumhydroxid, Alkalicarbonate wie Natrium-, Kalium- der Cäsiumcarbonat oder Natrium-, Kalium- oder Cäsiumhydrogencarbonat, Pyridin oder 4-Dimethylaminopyridin, Alkalihydride wie Lithium-, Natrium- oder Kaliumhydrid, Alkaliamide wie Natrium- und Kaliumamid, ferner Natrium- oder Kalium-tert.-butoxid, Lithium-, Natrium- oder Kalium-triphenylmethyl und Naphthalinlithium, -natrium oder -kalium. Es können aber auch andere übliche Basen verwendet werden.

Als Reaktionsbeschleuniger kommen vorzugsweise Metallhalogenide wie Natriumjodid, oder Kaliumjodid, quaternäre Ammoniumsalze wie Tetrabutylammoniumchlorid, -bromid, -jodid oder -hydrogensulfat, Benzyltriethylammoniumchlorid oder -bromid oder Kronenether wie 12-Krone-4, 15-Krone-5, 18-Krone-6, Dibenzo-18-krone-6 oder Dicyclohexano-18-krone-6 in Frage.

Die Umsetzung wird im allgemeinen bei Temperaturen zwischen 10 und 150, insbesondere 20 bis 120°C, drucklos oder unter Druck, kontinuierlich oder diskontinuierlich durchgeführt.

Ist Me ein Metallatom, wird die Reaktion a) gegebenenfalls in Gegenwart eines Lösungs- oder Verdünnungsmittels und bei Temperaturen zwischen -10 und 150, insbesondere 0 bis 120, bevorzugt 20 bis 80°C durchgeführt. Bei Anwesenheit eines Lösungsmittels wird die Umsetzung zweckmäßigerweise beim Siedepunkt des jeweiligen Lösungsmittels durchgeführt.

Zu den bevorzugten Lösungs- und Verdünnungsmitteln gehören Amide wie Dimethylformamid, Diethylformamid, Dimethylacetamid, Diethylacetamid, N-Methylpyrrolidon, Hexamethyl-phosphortriamid, Sulfoxide wie Dimethylsulfoxid und schließlich Sulfolan.

Für die Reaktion b) kommen als Lösungs- und Verdünnungsmittel Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ester wie Essigsäureethylester, Essigsäuremethylester oder Essigsäurebutylester, Ether wie Tetrahydrofuran, Diethylether, Dimethoxyethan, Dioxan, Diisopropylether oder Methyl-tert.-butylether, Amide wie Dimethylformamid, Dimethylacetamid oder N-Methylpyrrolidon, ferner Dimethylsulfoxid, Sulfolan oder entsprechende Gemische in Frage.

Die neuen Ausgangsverbindungen II erhält man durch Epoxidierung der entsprechenden Olefine V:

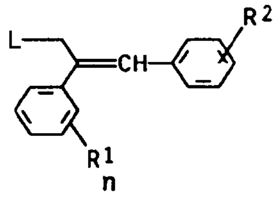

V

(vgl. G. Dittus in Houben-Weyl-Müller, Methoden der Organischen Chemie, Georg Thieme Verlag, Stuttgart, 1965, Bd. VI, 3, Seite 385 ff).

Die Verbindung V stellt man her, indem man Olefine der Formel VI

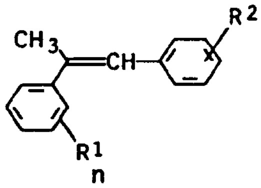

VI

nach bekannten Methoden in Allylposition halogeniert oder oxidiert.

Geeignete Halogenierungsreagenzien sind N-Chlor- und N-Bromsuccinimid. Als Lösungsmittel dienen z.B. halogenierte Kohlenwasserstoffe wie Tetrachlorkohlenstoff, Trichlorethan oder Methylenchlorid. Die Halogenierung wird i.a. bei Temperaturen zwischen 20 und 100°C vorgenommen.

Zur Allyloxidation verwendet man Perester wie Perbenzoesäure-tert.-butylester oder Peressigsäure-tert.-butylester in Anwesenheit eines Schwermetallsalzes wie z. B. Kupfer-I-chlorid oder Kupfer-I-bromid. Die Oxidation wird in der Regel in inerten Lösungsmitteln wie Dichlormethan, Toluol, Xylol, Chloroform, Tetrachlormethan oder Dichlormethan bei Temperaturen zwischen 10 und 100°C durchgeführt.

Die so erhaltenen Allylhalogenide V mit L = Halogen bzw. auch die Allylalkohole mit L = OH werden anschließend in die entsprechenden Epoxide II und VII überführt.

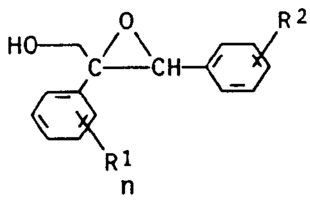

VII

Dazu oxidiert man die Olefine V mit Peroxycarbonsäuren wie Perbenzoesäure, 3-Chlorperbenzoesäure, 4-Nitroperbenzoesäure, Monoperphthalsäure, Peressigsäure, Perpropionsäure, Permaleinsäure, Monoperbernsteinsäure, Perpelargonsäure oder Trifluorperessigsäure in indifferenten Lösungsmitteln, vorzugsweise chlorierte Kohlenwasserstoffe, z. B. Methylenchlorid, Chloroform, Tetrachlorkohlenstoff, Dichlorethan, aber gegebenenfalls auch in Essigsäure, Essigester, Aceton oder Dimethylformamid, gegebenenfalls in Gegenwart eines Puffers wie Natriumacetat, Natriumcarbonat, Dinatriumhydrogenphosphat oder Triton B.

Man führt die Umsetzung bei Temperaturen zwischen 10 und 100 °C durch und katalysiert die Reaktion gegebenenfalls z. B. mit Jod, Natriumwolframat oder Licht. Zur Oxidation eignen sich auch alkalische Lösungen

von Wasserstoffperoxid (ca. 30 %ig) in Methanol, Ethanol, Aceton oder Acetonitril bei 25 bis 30 °C sowie Alkylhydroperoxide, z. B. tert.-Butylhydroperoxid, Cumolhydroperoxid und Cyclohexylhydroperoxid, gegebenenfalls unter Zusatz eines Katalysators, z. B. Natriumwolframat, Perwolframsäure, Molybdänhexacarbonyl oder Vanadylacetylacetonat. Die genannten Oxidationsmittel lassen sich z. T. in situ erzeugen.

Während die so erhaltenen Epoxihalogenide II (L = Halogen) gemäß Verfahren a) sofort umgesetzt werden können, überführt man die entsprechenden Epoxialkohole VII in reaktive Ester, die dann mit den Verbindungen III gemäß Verfahren a) umgesetzt werden.

Die Darstellung der reaktiven Ester, die mit III umgesetzt werden, erfolgt nach allgemein bekannten Methoden (Houben-Weyl-Müller, Methoden der Organischen Chemie, Georg Thieme Verlag, Stuttgart, 1955, Band 9, Seiten 388, 663, 671). Solche Ester sind beispielsweise Methansulfonsäureester, Trifluormethansulfonsäureester, 2,2,2-Trifluorethansulfonsäureester, Nonafluorbutansulfonsäureester, 4-Methylbenzolsulfonsäureester, 4-Brombenzolsulfonsäureester, 4-Nitrobenzolsulfonsäureester oder Benzolsulfonsäureester.

Die Verbindungen V lassen sich entsprechend allgemein bekannten Verfahren zur Olefinsynthese (Houben-Weyl-Müller, Methoden der Organischen Chemie, Georg Thieme Verlag, Stuttgart, 1972, Band V, 1b) herstellen.

Weiterhin können die Epoxyalkohole VII durch Oxidation und anschließender Reduktion der nach DE-A 37 22 886 erhältlichen Propenale hergestellt werden. Diese substituierten Propenale werden in Alkoholen wie Methanol, Ethanol, n- oder iso-Propanol oder Butanole gelöst, oder man verwendet Lösungsmittelgemische, die diese Alkohole enthalten, und oxidiert unter Zugabe von Basen mit Wasserstoffperoxid, Cyclohexylperoxid, tert.-Butylhydroperoxid oder Cumolhydroperoxid. Das erhaltene Formyloxiran kann ohne weitere Reinigung mit basischer Natriumborhydridlösung oder katalytisch zum entsprechenden Epoxyalkohol VII reduziert werden.

Die nachfolgenden Beispiele erläutern die Herstellung der Wirkstoffe:

## Herstellungsbeispiele

### I Herstellung der Ausgangsstoffe

#### Beispiel A

Zu einer Lösung von 40 g 2-Trifluormethylbenzaldehyd in 300 ml Methanol werden 4,2 g Natriumhydroxid in 30 ml Wasser gegeben. Das Reaktionsgemisch wird auf 10 °C gekühlt und schnell 35 g Phenylacetaldehyd zugesetzt, wobei die Temperatur in der Lösung auf 30-40 °C ansteigt. Nach 10stündigem Rühren bei 40 °C wird der farblosen Reaktionslösung 200 ml Wasser zugesetzt und die nun entstandene Emulsion mit Methyl-tert.-butylether ausgeschüttelt. Die organische Phase wird abgetrennt, über Natriumsulfat getrocknet und eingeengt. Nach Säulenfiltration des verbleibenden Rückstandes an Kieselgel (Essigester/n-Hexan = 1:3) erhält man in 80 %ger Ausbeute das E/Z-2-Phenyl-3-(2-trifluormethylphenyl)-propenal.

#### Beispiel B

52 g E/Z-2-Phenyl-3-(2-trifluormethylphenyl)-propenal werden in 300 ml Methanol gelöst und 2,2 ml konzentrierte Natronlauge zugesetzt. Die Reaktionslösung wird bei 0 °C gerührt, während 14,3 g Wasserstoffperoxid (ca. 50%ig) langsam zugetropft werden, wobei die Innentemperatur von 30 °C nicht überschritten wird. Nach beendeter Zugabe wird 6 Stunden bei Raumtemperatur gerührt und anschließend 2,35 g Natriumborhydrid zugegeben, das in wenig 10%iger Natronlauge gelöst ist. Nachdem das Reaktionsgemisch 18 Stunden bei Raumtemperatur gerührt wurde, setzte man Lösung 200 ml Wasser zu und schüttelte die entstandene Emulsion mit Methylenchlorid aus. Die organische Phase wird daraufhin über Natriumsulfat getrocknet, ein geengt und der verbleibende Rückstand aus Isopropanol umkristallisiert. Man erhält in 62%iger Ausbeute cis-2-Hydroxymethyl-2-phenyl-3-(2-trifluoromethylphenyl)-oxiran.

#### Beispiel C

Zu einer Lösung von 49 g cis-2-Hydroxymethyl-2-phenyl-3-(2-trifluoromethylphenyl)-oxiran in 200 ml Dichlormethan und 53 g Triethylamin werden bei Raumtemperatur 37,5 g 4-Methylbenzolsulfonsäurechlorid zugesetzt. Nach 24 Stunden wird das Reaktionsgemisch mit wäßriger Natriumhydrogencarbonat-Lösung und Wasser gewaschen, über Natriumsulfat getrocknet und im Vakuum eingedampft. Aus dem Rückstand erhält man 55 g cis-2-(4-Methylphenylsulfonyl-oxy-methyl)-2-phenyl-3-(2-trifluorphenyl)-oxiran, das anschließend mit Triazol gemäß folgendem Beispiel weiterverarbeitet wird.

II Herstellung der Endprodukte

Beispiel 1

Eine Lösung von 9,4 g 1,2,4-Triazol in 100 ml N-Methylpyrrolidon wird mit 5,2 g Natriumhydroxid versetzt und für 30 Minuten auf 50 °C erwärmt. Nachdem das Reaktionsgemisch auf Raumtemperatur gekühlt wurde, wird der Lösung 57 g cis-2-(4-Methylphenylsulfonyloxymethyl)-2-phenyl-3-(2-trifluormethylphenyl)-oxiran, das in 100 ml N-Methylpyrrolidon gelöst ist, langsam zugetropft und 12 Stunden bei Raumtemperatur gerührt. Anschließend wird 200 ml Wasser zugegeben und mehrmals mit Methyl-tert-butylether extrahiert; die organische Phase zweimal mit Wasser gewaschen, über Natriumsulfat getrocknet und eingeengt. Man erhält durch Kristallisation aus Methyl-tert.-butylether/n-Hexan in 75 %iger Ausbeute cis-2-(1,2,4-Triazol-1-yl-methyl)-2-phenyl-3-(2-trifluormethylphenyl)-oxiran mit dem Schmelzpunkt 132-134 °C (Verbindung Nr. 1).

Entsprechend Beispiel 1 können die in der Tabelle aufgeführten Verbindungen hergestellt werden:

Tabelle

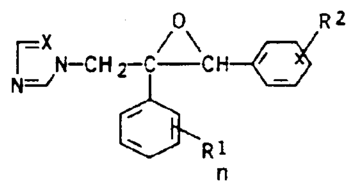

I

| Nr. | $R^1_n$ | $R^2$ | X | Schmp. (°C)/IR |
|---|---|---|---|---|
| 1 | H | $2-CF_3$ | N | 132-134 |
| 2 | 2-Cl | $2-CF_3$ | N | 1506, 1315, 1172, 1124, 1038, 771 $cm^{-1}$ |
| 3 | 2-F | $2-CF_3$ | N | |
| 4 | 4-Cl | $2-CF_3$ | N | 117-119 |
| 5 | 4-F | $2-CF_3$ | N | 110-112 |
| 6 | $2,4-F_2$ | $2-CF_3$ | N | |
| 7 | $2,4-Cl_2$ | $2-CF_3$ | N | |
| 8 | 4-Br | $2-CF_3$ | N | |
| 9 | $4-OCF_3$ | $2-CF_3$ | N | |
| 10 | $4-OCH_3$ | $4-CF_3$ | N | |
| 13 | 2-F | $4-CF_3$ | N | |
| 15 | 4-F | $4-CF_3$ | N | |
| 16 | $2,4-F_2$ | $4-CF_3$ | N | |
| 18 | 4-Br | $4-CF_3$ | N | |
| 19 | $4-OCF_3$ | $4-CF_3$ | N | |

*) 2:1-cis/trans-Gemisch

Die Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Hemileia vastatrix an Kaffee,
Alternaria solani an Kartoffeln, Tomaten,
Sclerotium rolfsii an Erdnüssen und Rasen-Arten,
Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, benzol), chlorierte Aromaten (z.B. Chlorbenzole), Paraffine (z.b. Erdölfraktionen), Alkohole (z.B. Methanol, butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden. Bei der Anwendung der Wirkstoffe im Materialschutz u.a. zur Bekämpfung holzzerstörender Pilze wie Coniophora puteana und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, beizen oder Gießen.

Beispiele für solche Zubereitungen sind:

I. Man vermischt 90 Gew.-Teile der Verbindung Nr. 5 mit 10 Gew.-Teilen N-Methyl-α-pyrrolidon und erhält eine Lösung, die zur Anwendung in Form kleinster Tropfen geeignet ist.

IV. 20 Gew.-Teile der Verbindung Nr. 5 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 1 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VII.30 Gew.-Teile der Verbindung Nr. 1 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 5 werden mit 10 Gew.-Teilen Natriumsalz eines Phenolsulfonsäureharnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Für die folgenden Versuche wurde der Aus EP-A 94 564 bekannte Wirkstoff 2-(1,2,4-Triazolyl-1-yl-methyl)-2-(4-chlorphenyl)-3-(3-trifluormethylphenyl)oxiran (A) verwendet.

Anwendungsbeispiel

Wirksamkeit gegen Botrytis cinerea an Paprika

Paprikasämlinge der Sorte "Neusiedler Ideal Elite" wurden, nachdem sich 4 bis 5 Blätter gut entwickelt hatten, mit wäßrigen Suspensionen, die 80 % Wirkstoff und 20 % Emulgiermittel in der Trockensubstanz enthielten, tropfnaß gespritzt. Nach dem Antrocknen des Spritzbelages wurden die Pflanzen mit einer Konidienaufschwemmung des Pilzes Botrytis cinerea besprüht und in eine Kammer mit hoher Luftfeuchtigkeit bei 22 bis 24°C gestellt. Nach 5 Tagen hatte sich die Krankheit auf den unbehandelten Kontrollpflanzen so stark entwickelt, daß die entstandenen Blattnekrosen den überwiegenden Teil der Blätter bedeckten.

Das Ergebnis zeigt, daß die Wirkstoffe Nr. 1 und 5 bei der Anwendung als 0,05 %ige (Gew.-%) Spritzbrühe eine bessere fungizide Wirkung zeigen (0 - 10 % Befall) als das Vergleichsprodukt A (80 % Befall).

## Patentansprüche

1. Verfahren zur Bekämpfungfung von Pilzen, dadurch gekennzeichnet, daß man eine fungizid wirksame Menge eines Azolylmethyloxirans der allgmeinen Formel I

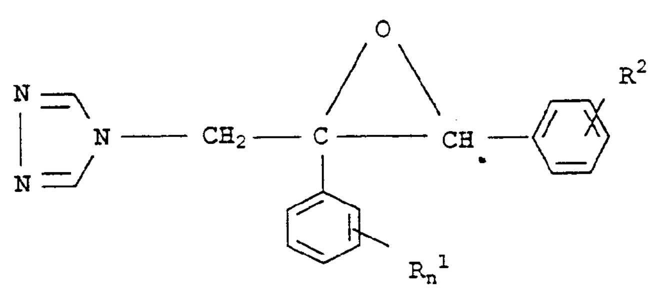

in der
$R^1$ Wasserstoff, Halogen, Nitro, Phenyl, Phenoxy, $C_1$-$C_4$-Alkyl, $C_1$-$C_5$-Alkoxy, $C_1$-$C_4$-Halogenalkyl, $C_1$-$C_5$-Halogenalkoxy und darüber hinaus $R^1$ zusammen mit dem Phenylkern auch einen Naphthylrest bilden kann, der unsubstituiert oder durch $R^1$ substituiert ist,
n eine ganze Zahl von 1 bis 5,
$R^2$ ortho-Trifluormethyl,
bedeuten, oder der Verbindungen der Formel I, in der $R^1$ Fluor, Brom, Nitro, Phenyl, Phenoxy, $C_1$-$C_5$-Alkyl, $C_1$-$C_5$-Alkoxy, $C_1$-$C_4$-Halogenalkyl, $C_1$-$C_5$-Halogenalkoxy und darüber hinaus $R^1$ zusammen mit dem Phenylkern auch einen Naphthylrest bilden kann, der unsubstituiert oder durch $R^1$ substituiert ist,
n eine ganze Zahl von 1 bis 5,
$R^2$ Para-Trifluormethyl,
bedeuten, oder deren für Pflanzen verträgliche Säureadditions- oder Metallsalze auf die Pilze oder auf durch Pilzbefall bedrohte Materialien, Flächen, Hölzer, Pflanzen oder Saatgüter einwirken läßt.

2. Verfahren gemäß Anspruch 1, dadurch gekennzeichnet, daß man eine Verbindung der Formel I gemäß Anspruch 1 verwendet, in der $R^1$ Wasserstoff und $R^2$ 2-$CF_3$ bedeutet.

3. Verfahren gemäß Anspruch 1, dadurch gekennzeichnet, daß man eine Verbindung der Formel I gemäß Anspruch 1 verwendet, in der $R^1$ 4-Fluor und $R^2$ 2-$CF_3$ bedeutet.

## Claims

1. A method for controlling fungi, which comprises a fungicidally effective amount of an azolylmethyloxirane of the formula I

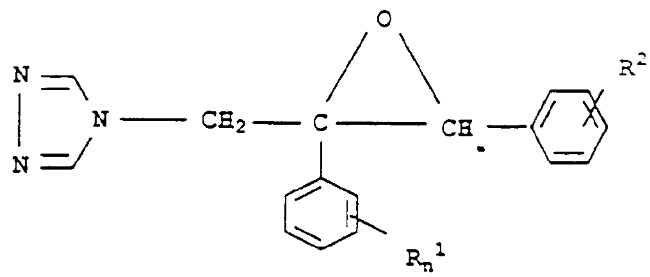

where
$R^1$ is hydrogen, halogen, nitro, phenyl, phenoxy, $C_1$-$C_5$-alkyl, $C_1$-$C_5$-alkoxy, $C_1$-$C_4$-haloalkyl, $C_1$-$C_5$-haloalkoxy and, furthermore, $R^1$ together with the phenyl nucleus may also form naphthyl which is unsubstituted or substituted by $R^1$,
n is an integer from 1 to 5,
$R^2$ is ortho-trifluoromethyl,

or of a compound of the formula I where $R^1$ is fluorine, bromine, nitro, phenyl, phenoxy, $C_1$-$C_5$-alkyl, $C_1$-$C_5$-alkoxy, $C_1$-$C_4$-haloalkyl, $C_1$-$C_5$-haloalkoxy and, furthermore, $R^1$ together with the phenyl nucleus may also form naphthyl which is unsubstituted or substituted by $R^1$,
n is an integer from 1 to 5,
$R^2$ is para-trifluoromethyl,
or a plant-tolerated acid addition salt or metal salt thereof being allowed to act on the fungi, or the materials, areas, timber, plants or seed threatened by fungus attack.

2. A method as claimed in claim 1, wherein a compound of the formula I is as claimed in claim 1, where $R^1$ is hydrogen and $R^2$ is 2-$CF_3$, is used.

3. A method as claimed in claim 1, wherein a compound of the formula I as claimed in claim 1, where $R^1$ is 4-fluorine and $R^2$ is 2-$CF_3$, is used.

## Revendications

1. Procédé de lutte contre les champignons, caractérisé par le fait que l'on fait agir sur les champignons ou les matériaux, surfaces, bois, plantes ou semences menacés par l'attaque par les champignons une quantité efficace au point de vue fongicide d'une azolylméthyloxirane de formule générale I

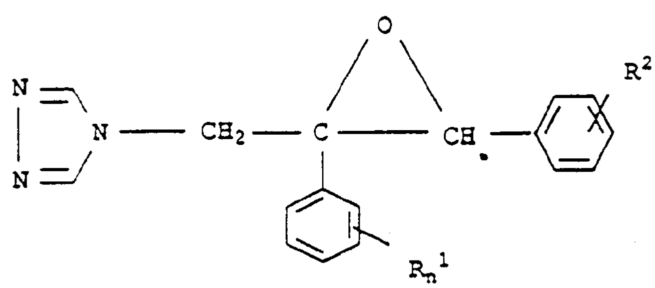

dans laquelle
$R^1$ représente hydrogène, halogène, nitro, phényle, phénoxy, alkyle en $C_1$-$C_5$, alcoxy en $C_1$-$C_5$, halogènalkyle en $C_1$-$C_4$, halogènalcoxy en $C_1$-$C_5$, $R^1$ pouvant en outre former ensemble avec le noyau phényle, un reste naphtyle, non substitué ou substitué par $R^1$,
n, un nombre entier de 1 à 5,
$R^2$, ortho-trifluorométhyle,
ou de composés de formule I dans laquelle $R^1$ représente fluor, brome, nitro, phényle, phénoxy, alkyle en $C_1$-$C_5$,

alcoxy en $C_1$-$C_5$, halogènalkyle en $C_1$-$C_4$, halogènalcoxy en $C_1$-$C_5$, $R^1$, ensemble avec le noyau phényle, pouvant aussi former un reste naphtyle, non substitué ou substitué par $R^1$,
n, un nombre entier de 1 à 5,
$R^2$, para-trifluorométhyle,
ou de leurs sels d'addition d'acide ou sels métalliques tolérés par les plantes.

2. Procédé selon la revendication 1, caractérisé par le fait que l'on utilise un composé de formule I, selon la revendication 1, dans laquelle $R^1$ représente hydrogène et $R^2$, 2-$CF_3$.

3. Procédé selon la revendication 1, caractérisé par le fait que l'on utilise un composé de formule I, selon la revendication 1, dans laquelle $R^1$ représente 4-fluor et $R^2$, 2-$CF_3$.